# EUROPEAN PATENT APPLICATION

(11) **EP 4 282 379 A1**
(43) Date of publication of application: **29.11.2023**
(21) Application number: 22752089.7
(22) Date of filing: 18.01.2022
(51) Int. Cl.: A61C 19/06, A61B 34/20, A61B 90/00

(54) **SURGICAL MICROSCOPE DIAGNOSIS AND TREATMENT SYSTEM**

(30) Priority: 10.02.2021 CN 202110185546
(71) Applicant: Zumax Medical Co., Ltd., Jiangsu 215129 (CN)
(72) Inventor: WANG, Jilong, Suzhou, Jiangsu 215129 (CN); NADEAU, Bobby, Suzhou, Jiangsu 215129 (CN); LI, Jianyue, Suzhou, Jiangsu 215129 (CN); HUANG, Bin, Suzhou, Jiangsu 215129 (CN); DU, Lei, Suzhou, Jiangsu 215129 (CN); HE, Jin, Suzhou, Jiangsu 215129 (CN)
(74) Representative: Sun, Yiming
(86) International application number: PCT/CN2022/072540
(87) International publication number: WO 2022/170927

(57) **Abstract**

A surgical microscope diagnosis and treatment system comprises a microscopic observation module, a storage module, and an enhanced information image injection module. The microscopic observation module is configured to observe a target object to be observed. The storage module stores a radiatively imaged three-dimensional structure digital image of the target object. The enhanced information image injection module is used for projecting the radiatively imaged three-dimensional structure digital image into an observation field of the microscopic observation module in a manner of an optical image, so as to be superimposed on an optical image under a microscope in the observation field of the microscopic observation module to form a superimposed optical image. According to the surgical microscope diagnosis and treatment system, an additional information image can be superimposed on the optical image under the microscope in a projection manner, so that an operator can conveniently and flexibly observe the target object during a surgery, and can also quickly view a variety of data information related to the surgery.

## Description

### Field of the Invention

The present disclosure relates to the technical field of dental diagnosis and treatment, and in particular to a surgical microscope diagnosis and treatment system.

### Background technology

A surgical microscope can be used in examination and treatment of pulp and root canals. With the surgical microscope, the operator can clearly observe the position of a root canal orifice, the form of the inner wall of a root canal and the removal of the pulp in the root canal, prepare for and fill the root canal, take out broken instruments in the root canal and perform a periapical surgery. With sufficient illumination and the ability of providing clear magnified images, the popularity of the dental surgical microscope has changed the traditional crude operation based on experience and hand feeling, and the success rate of root canal treatment, removal of metal blockage in the root canal, and treatment of root canal steps, apical deviation and pulp cavity perforation is greatly improved.

Because of the need to accommodate the patient's position, the doctor is unable to maintain a normal and comfortable posture. Continuous fine operations will cause muscular fatigue and soreness in the shoulders, neck and back, which can accumulate over time and lead to serious joint problems and even affect the doctor's professional life. The emergence of the surgical microscope has enabled the above problems to be solved. The surgical microscope enables doctors to maintain an ergonomically correct posture during examination and treatment, eliminates fatigue in the shoulders, neck and back, and effectively improves the efficiency and quality of diagnosis and treatment.

With the traditional surgical microscope, the doctor can only observe the structure of the outer layer of the tissues, but cannot observe the structure of the internal tissues, and some operations require repeated trials, which takes a long time and is likely to miss the root canal or remove too much healthy tooth tissue. A tooth is a three-dimensional structure with multi-layered tissues. With the surgical microscope, the doctor can only observe detailed features of the outer layer of tissues, but cannot determine the structure of the internal tissues.

The cavity in the middle of the tooth contains a soft tissue called pulp. The cavity has a wide upper part called pulp cavity, and the lower part has a tubular root canal, from which blood vessels of dental nerves and trophic nerves are derived. Infection of pulp will cause pain and jaw infection, and finally the tooth will become fragile because of the death of the dental nerves.

Taking root canal treatment as an example, the doctor needs to completely open the pulp cavity, find all root canals and deal with them. Humans generally have 1 to 4 root canals per tooth, with the most root canals in the back teeth. In a tooth with multiple root canals, it is always difficult to find the root canal orifice due to age-related changes, deposition of reparative dentin, or pulp stone, or calcification of pulp cavity, or changes in the form of the root canal. In this case, it is necessary to understand and check the anatomic form of the pulp cavity from various directions and positions by the aid of a three-dimensional anatomic form of the tooth, and to know and point out, based on X-ray films taken by a multi-angle projection method, the number, shape, position, direction and bending of roots and root canals, the relationship between the root and crown, and various possible variations of the anatomic forms of the root and root canal. Some teeth may have up to four root canals, and there may be complicated situations such as lateral root canals, accessory root canals, apical ramifications and apical furcation, which may be missed even under magnification observation. When it is required to estimate the possible position of the root canal, if necessary, a small amount of dentin can be removed by a small ball drill from the developmental groove where the root canal may be or is expected to be located, then a sharp probe is used to try to pierce any calcified area to point out the root canal orifice, and the ferrule of the dental neck is removed to expose the position of the root canal orifice. That is, if there is calcification at the root canal orifice, it is more necessary for the doctor to repeatedly try various possible positions, so it is inevitable to remove too much healthy tooth tissue.

At present, preoperative dental films are often used to help the doctor judge and determine the number and form of root canals. First, the doctor needs to take effort to remember the form of root canals, or even pause the surgery and observe the dental film again. Besides, since the dental film is only a two-dimensional plane image, it cannot accurately reflect the three-dimensional form of the root canals. In fact, many root canals have multiple bends, and it is impossible to accurately locate the root canals by the dental film.

Three-dimensional images of CBCT are complex and difficult to remember. The doctors need to memorize the three-dimensional form of the tooth structure in their brains, and compare, superimpose and fuse it with the real object under the microscope, which requires a lot of effort. Moreover, it is difficult to ensure the accuracy and precision.

Therefore, in view of the above technical problems, it is necessary to provide a new technical solution.

### SUMMARY OF THE INVENTION

In order to solve the technical problems in the prior art, this application provides a surgical microscope diagnosis and treatment system. The specific solution is as follows:
A surgical microscope diagnosis and treatment system comprises a microscopic observation module, a storage module and an enhanced information image injection module. The microscopic observation module is configured to observe a target object to be observed. The storage module stores a radiatively imaged three-dimensional structure digital image of the target object. The enhanced information image injection module is configured to project the radiatively imaged three-dimensional structure digital image into an observation field of the microscopic observation module in a manner of an optical image, so as to be superimposed on an optical image under a microscope in the observation field of the microscopic observation module to form a superimposed optical image.

Further, the radiatively imaged three-dimensional structure digital image is controllable to be on or off, so that an operator chooses to view a layered two-dimensional image or a 3D image according to needs.

Further, the surgical microscope diagnosis and treatment system comprises a 3D imaging module and an image recognition and processing module. The 3D imaging module is configured to collect the optical image under the microscope in the observation field of the microscopic observation module in real time and convert the optical image under the microscope into a three-dimensional digital image. The image recognition and processing module is configured to recognize biological characteristics in the three-dimensional digital image, perform comparison of biological characteristics and transmit the radiatively imaged three-dimensional structure digital image matched with the three-dimensional digital image to the enhanced information image injection module, and then the radiatively imaged three-dimensional structure digital image is projected into a set area in the observation field of the microscopic observation module.

Further, the surgical microscope diagnosis and treatment system further comprises a detection module. A zoom large objective lens and a zoom system are arranged in the microscopic observation module. The detection module is configured to respectively detect a focusing position of the zoom large objective lens and a magnification of the zoom system. The image recognition and processing module determines a depth position of the radiatively imaged three-dimensional structure digital image according to the focusing position of the zoom large objective lens detected by the detection module. The image recognition and processing module determines a depth range of a current layer area displayed by the radiatively imaged three-dimensional structure digital image according to the magnification of the zoom system detected by the detection module.

Further, the radiatively imaged three-dimensional structure digital image is projected to an edge of the observation field of the microscopic observation module, or the radiatively imaged three-dimensional structure digital image is displayed in superposition with the optical image under the microscope in the observation field of the microscopic observation module, and a transparency of the radiatively imaged three-dimensional structure digital image is adjustable.

Further, the surgical microscope diagnosis and treatment system further comprises a positioning and navigation-based detection module. The positioning and navigation-based detection module is mounted on a surgical instrument. The positioning and navigation-based detection module is configured for real-time detection of depth and spatial position data of the surgical instrument. The image recognition and processing module compares the depth and spatial position data collected by the positioning and navigation-based detection module with the biological characteristics in the three-dimensional digital image to obtain real-time relative position data between the surgical instrument and the target object, and transmits the real-time relative position data to the enhanced information image injection module, and then the real-time relative position data is projected to a set position of the observation field of the microscopic observation module.

Further, the depth and spatial position data of the surgical instrument and state data of the surgical instrument are stored in the storage module in real time, and the state data of the surgical instrument is capable of being transmitted to the enhanced information image injection module and then projected to the set position of the observation field of the microscopic observation module.

Further, the storage module also stores patient information data, apex locator data, intraoral scanner data, electronic periodontal probe data and pulp vitality data, and each data is respectively controllable to be on or off, so that an operator projects required data to a set position of the observation field of the microscopic observation module according to needs by means of the enhanced information image injection module.

Further, each data is projected to the set position of the observation field of the microscopic observation module in a manner of text and symbols, data tables, two-dimensional curves or three-dimensional topographic maps. Each data is displayed in a split screen in the observation field of the microscopic observation module or switched to display in a same window, and a display transparency of each data and a size and a position of the display window of each data are adjustable.

Further, the surgical microscope diagnosis and treatment system further comprises an Al auxiliary analysis module. The Al auxiliary analysis module is controllable to be on or off, so that the operator chooses to turn on or off an Al auxiliary function according to needs. The Al auxiliary analysis module is configured to analyze a three-dimensional digital image collected by a 3D imaging module, identify lesions of the target object and leave a mark or reminder on the target object according to the lesions, and also configured to comprehensively analyze all the data stored in the storage module to generate additional Al auxiliary information, and then the Al auxiliary information is projected to the set position of the observation field of the microscopic observation module by means of the enhanced information image injection module.

Further, the surgical microscope diagnosis and treatment system further comprises a camera module. The camera module is configured to collect expression image data of a patient. The Al auxiliary analysis module analyzes the expression image data collected by the camera module, determines a comfort level of the patient, and projects the real-time projection to the set position of the observation field of the microscopic observation module by means of the enhanced information image injection module.

Further, the microscopic observation module comprises a surgical microscope. The enhanced information image injection module comprises a projection apparatus. The projection apparatus is capable of projecting an additional information beam. The additional information beam and one or two incident beams in the surgical microscope are superimposed and then enter a binocular tube of the surgical microscope to form the superimposed optical image.

Further, the projection apparatus comprises a projection display unit and an imaging lens set. The projection display unit is capable of projecting an additional optical image, and the additional optical image is converted into parallel beams by the imaging lens set to form the additional information beam.

Compared with the prior art, the surgical microscope diagnosis and treatment system of this application has one or more of the following beneficial effects:
(1) According to the surgical microscope diagnosis and treatment system of this application, some auxiliary additional information images are superimposed on the optical image under the microscope in the field of the microscopic observation by means of the enhanced information image injection module, so that the user can quickly view multiple data information related to the surgery during the surgery process.
(2) According to the surgical microscope diagnosis and treatment system of this application, the radiatively imaged three-dimensional structure digital image is controllable to be on or off, so that the operator can retrieve and view the 3D structure of the target object at any time, and choose to view the specific layered image so as to determine the internal structure of the tissue under the microscope.
(3) According to the surgical microscope diagnosis and treatment system of this application, the image recognition and processing module can automatically compare and register the radiatively imaged three-dimensional structure digital image with the three-dimensional digital image of the optical image under the microscope by means of determination of biological characteristics.
(4) According to the surgical microscope diagnosis and treatment system of this application, the radiatively imaged three-dimensional structure digital image can be displayed in superposition with the optical image under the microscope, the positioning and navigation-based detection module is arranged in the surgical instrument to guide the surgical operation to be in place accurately, so that the operator can confirm the operation in time, which improves the efficiency.
(5) According to the surgical microscope diagnosis and treatment system of this application, the patient information, apex locator data, intraoral scanner data, electronic periodontal probe data and pulp vitality data can be displayed in the set position of the observation field of the microscopic observation module, which is convenient for providing reference for the operator.
(6) According to the surgical microscope diagnosis and treatment system of this application, the Al auxiliary module can realize Al auxiliary diagnosis and display.

### BRIEF DESCRIPTION OF THE DRAWINGS

FIG. 1 is a schematic structural view of a surgical microscope according to Embodiment I of this embodiment;
FIG. 2 is a schematic view of superimposition of optical paths of the surgical microscope according to Embodiment I of this embodiment;
FIG. 3 is a schematic view of superimposition of optical paths of a surgical microscope according to Embodiment II of this embodiment;
FIG. 4 is a schematic view of superimposition of optical paths of a surgical microscope according to Embodiment III of this application;
FIG. 5 is a schematic view of superimposition of optical paths of a surgical microscope according to Embodiment IV of this application;
FIG. 6 is a schematic view of superimposition of optical paths of a surgical microscope according to Embodiment V of this application;
FIG. 7 is a schematic view of superimposition of optical paths of a surgical microscope according to Embodiment VI of this application;
FIG. 8 is a schematic view of superimposition of optical paths of a surgical microscope according to Embodiment VII of this application;
FIG. 9 is a schematic view of superimposition of optical paths of a surgical microscope according to Embodiment VIII of this application;
FIG. 10 is an effect picture of superimposition of optical paths of a surgical microscope according to Embodiment IX of this application;
FIG. 11 is a flowchart of a surgical microscope diagnosis and treatment system according to Embodiment IX of this embodiment;
FIG. 12 is a flowchart of a surgical microscope diagnosis and treatment system according to Embodiment X of this embodiment;
FIG. 13 is a flowchart of a surgical microscope diagnosis and treatment system according to Embodiment XI of this embodiment;
FIG. 14 is a flowchart of a surgical microscope diagnosis and treatment system according to Embodiment XII of this embodiment; and
FIG. 15 is a flowchart of a surgical microscope diagnosis and treatment system according to Embodiment XIII of this embodiment.

Wherein, 1-microscope body, 2-zoom lens set, 3-superimposition lens set, 301-first longitudinal beam splitter prism, 302-first transverse beam splitter prism, 303-second transverse beam splitter prism, 304-first reflector, 305-second longitudinal beam splitter prism, 306-third longitudinal beam splitter prism, 307-second reflector, 308-third reflector, 309-fourth longitudinal beam splitter prism, 310-third transverse beam splitter prism, 311-fourth transverse beam splitter prism, 312-fifth transverse beam splitter prism, 4-large objective lens set, 5-binocular tube, 51-eyepiece, 6-projection apparatus, 61-projection display unit, 62-imaging lens set, 63-deflecting prism set, 64-housing, 7-image device, 8-filter apparatus, 91-first incident beam, 92-second incident beam, 93-additional optical image, 94-additional information beam, 95-first superimposed beam, 96-second superimposed beam, 97-third superimposed beam, 98-fourth superimposed beam, 99-fifth superimposed beam.

### DETAILED DESCRIPTION OF THE PREFERRED

In order to further explain the technical means and effects adopted by the present invention to achieve the intended objectives of the present invention, the specific implementations, structures, features and effects proposed according to the present invention will be described in detail below in conjunction with the accompanying drawings and preferred embodiments.

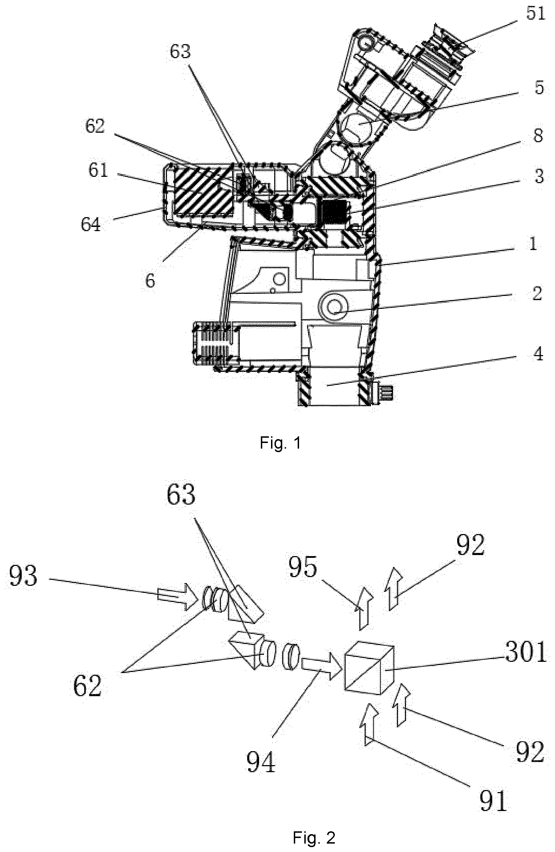

This application provides a surgical microscope, comprising a microscope body 1, a zoom lens set 2 and a superimposition lens set 3 built in the microscope body 1, and a large objective lens set 4, a binocular tube 5 and a projection apparatus 6 that are connected to the microscope body 1, as shown in FIG. 1. The surgical microscope is designed with two optical paths. The zoom lens set 2 comprises a first zoom lens set and a second zoom lens set. A first incident beam 91 sequentially passes through the large objective lens set 4 and the first zoom lens set and enters the binocular tube 5. A second incident beam 92 sequentially passes through the large objective lens set 4 and the second zoom lens set and enters the binocular tube 5. The projection apparatus 6 projects an additional information beam 94, and the additional information beam 94, and the first incident beam 91 and/or the second incident beam are superimposed by the superimposition lens set 3 and enter the binocular tube 5. The projection apparatus 6 comprises a projection display unit 61 and an imaging lens set 62. The projection display unit 61 is capable of projecting an additional optical image 93, and the additional optical image 93 is converted to parallel beams through the imaging lens set 62 to form the additional information beam 94. The projection display unit 61 is preferably a miniprojector.

### Embodiment I

The projection apparatus of this embodiment is mounted at a rear side of the microscope body 1. As shown in FIG. 1, the projection display unit 61 and the imaging lens set 62 are both located in a housing 64 of the projection apparatus 6, and additionally mounted as accessories between the surgical microscope body 1 and the binocular tube 5.

As shown in FIG. 2, the superimposition lens set 3 comprises a first longitudinal beam splitter prism 301. The projection display unit 61 projects the additional optical image 93 from the rear side of the microscope body 1. It should be noted that if the optical path projected by the projection display unit 61 is not the same as that of the first longitudinal beam splitter prism 301, in specific implementation, a deflecting prism set 63 may be added into the imaging lens set 62 as needed, so that after the additional optical image 93 projected by the projection display unit 61 is converted by the imaging lens set 62 to form the additional information beam 94, the additional information beam can directly enter the first longitudinal beam splitter prism 301. The deflecting prism set 63 used in the figure comprises two rectangular prisms. Of course, in specific implementation, the two rectangular prisms may also be replaced with rhomboid prisms, plane mirrors or the like. After the additional information beam 94 is superimposed with the first incident beam 91 through the first longitudinal beam splitter prism 301 to form a first superimposed beam 95, the first superimposed beam 95 passes through the binocular tube 5 to form an image, and the operator can observe the real optical image with additional information superimposed under the microscope through an eyepiece.

### Embodiment II

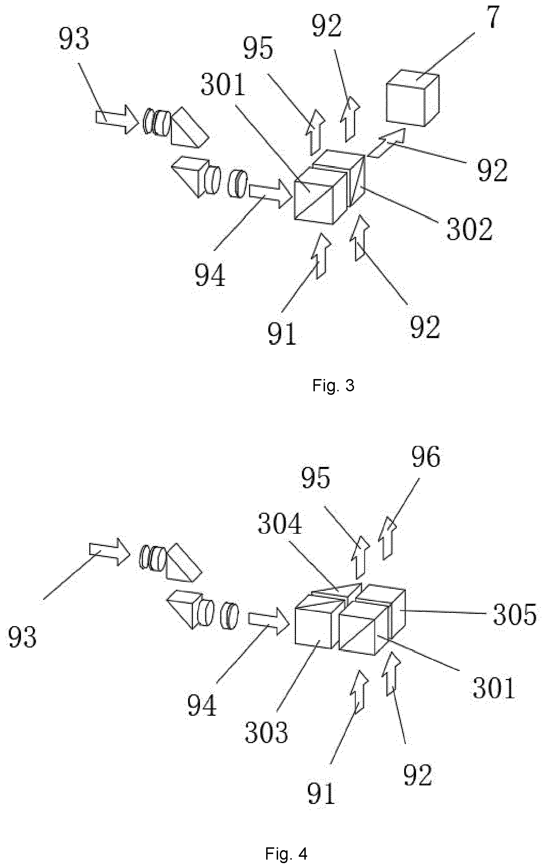

In this embodiment, on the basis of Embodiment I, in the superimposition lens set 3, a first transverse beam splitter prism 302 is added along an optical path of the second incident beam 92. As shown in FIG. 3, the second incident beam 92 passes through the second zoom lens set, and is split laterally by the first transverse beam splitter prism 302. One part of the second incident beam 92 continues to enter the binocular tube 5 along the original optical path, and the other part of the second incident beam 92 can be collected by an image device 7 so as to provide an image for the image device 7. The image device 7 is, for example, a camera, a video camera, a mobile phone, an assistant lens, etc.

### Embodiment III

In this embodiment, on the basis of Embodiment I, in the superimposition lens set 3, a second transverse beam splitter prism 303 is added between the imaging lens set 62 and the first longitudinal beam splitter prism 301, a second longitudinal beam splitter prism 305 is added along the optical path of the second incident beam 92, and a first reflector 304 is added between the second transverse beam splitter prism 303 and the second longitudinal beam splitter prism 305. As shown in FIG. 4, the additional information beam 94 is split laterally by the second transverse beam splitter prism 303. One part of the additional information beam 94 continues to enter the first longitudinal beam splitter prism 301 along the original optical path and is superimposed with the first incident beam 91 to form the first superimposed beam 95. The other part of the additional information beam 94 is deflected by 90° and reflected by the first reflector 304, and then enters the second longitudinal beam splitter prism 305 to be superimposed with the second incident beam 92 to form a second superimposed beam 96. Then, the first superimposed beam 95 and the second superimposed beam 96 passes through the binocular tube 5 to form an image, which can be observed through the eyepiece. In this embodiment, all beams entering the binocular tube 5 are superimposed beams.

### Embodiment IV

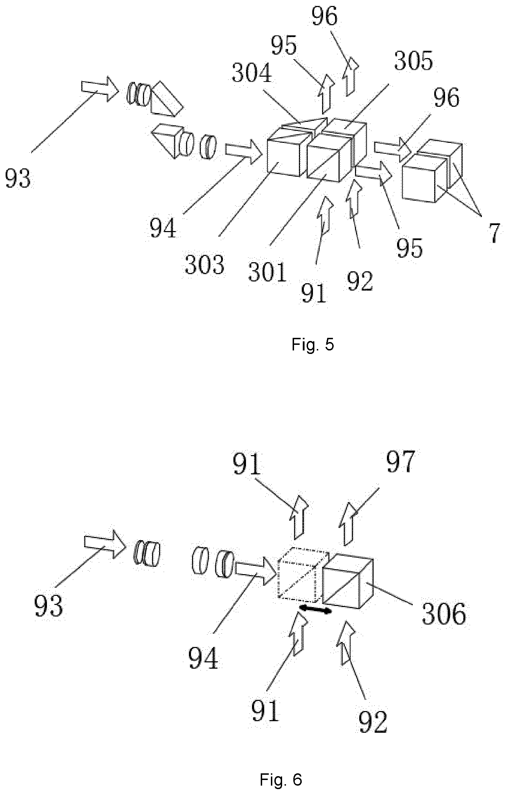

In this embodiment, on the basis of Embodiment III, lateral beam channels are respectively added to the microscope body 1 at positions corresponding to the first longitudinal beam splitter prism 301 and the second longitudinal beam splitter prism 305. As shown in FIG. 5, the first superimposed beam 95 is split laterally through the first longitudinal beam splitter prism 301. One part of the first superimposed beam 95 enters the binocular tube 5, and the other part of the first superimposed beam 95 exits through the corresponding lateral beam channel. The second superimposed beam 96 is split laterally through the second longitudinal beam splitter prism 305. One part of the second superimposed beam 96 enters the binocular tube 5, and the other part of the second superimposed beam 96 exits through the corresponding lateral beam channel. The first superimposed beam 95 and the second superimposed beam 96 exiting from the lateral beam channels can be collected by the image device 7 to provide an image for the image device 7. The image device 7 is, for example, a camera, a video camera, a mobile phone, an assistant lens, etc. In this case, the image obtained by the image device 7 is a superimposed image.

### Embodiment V

In this embodiment, the projection apparatus is mounted on the left side or right side of the microscope body 1. The projection display unit 61 projects the additional optical image 93 from the left side or right side of the microscope body 1. As shown in FIG. 6, the superimposition lens set 3 comprises a third longitudinal beam splitter prism 306. The third longitudinal beam splitter prism 306 can be driven to translate along a left-right direction of the microscope body 1 in the microscope body 1. The additional information beam 94 passes through the third longitudinal beam splitter prism 306 and is superimposed with the first incident beam 91 or the second incident beam 92 to form a third superimposed beam 97, and then the third superimposed beam 97 passes through the binocular tube 5 to form an image. The third longitudinal beam splitter prism 306 is designed to be capable of translating, so that the additional optical image 93 can be superimposed on the image of the surgical microscope along any optical path.

### Embodiment VI

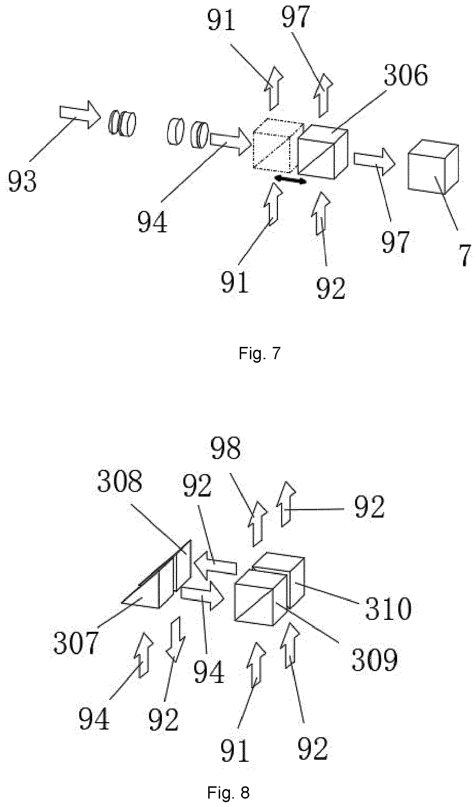

In this embodiment, on the basis of Embodiment V, a lateral beam channel is added to the microscope body 1 at the position corresponding to the third longitudinal beam splitter prism 306. As shown in FIG. 7, the third superimposed beam 97 is split laterally through the third longitudinal beam splitter prism 306. One part of the third superimposed beam 97 enters the binocular tube 5, and the other part of the third superimposed beam 97 exits through the corresponding lateral beam channel. The third superimposed beam 97 exiting from the lateral beam channel can be collected by the image device 7 so as to provide an image for the image device 7. The image device 7 is, for example, a camera, a video camera, a mobile phone, an assistant lens, etc. In this case, the image obtained by the image device 7 is a superimposed image.

### Embodiment VII

In this embodiment, the projection apparatus is mounted on the lower side or inside of the microscope body 1. The superimposition lens set 3 comprises a second reflector 307, a third reflector 308, a fourth longitudinal beam splitter prism 309 and a third transverse beam splitter prism 310. As shown in FIG. 8, the additional information beam 94 is reflected by the second reflector 307 and enters the fourth longitudinal beam splitter prism 309 from the rear side of the microscope body 1. The additional information beam 94 is superimposed with the first incident beam 91 through the fourth longitudinal beam splitter prism 309 to form a fourth superimposed beam 98. Then, the fourth superimposed beam 98 passes through the binocular tube 5 to form an image. The second incident beam 92 passes through the second zoom lens set and is split laterally by the third transverse beam splitter prism 310. One part of the second incident beam 92 continues to enter the binocular tube 5 along the original optical path, and the other part of the second incident beam 92 is reflected by the third reflector 308 and exits downward. The part of the second incident beam 92 exiting downward can be collected by the image device 7 so as to provide an image for the image device 7.

### Embodiment VIII

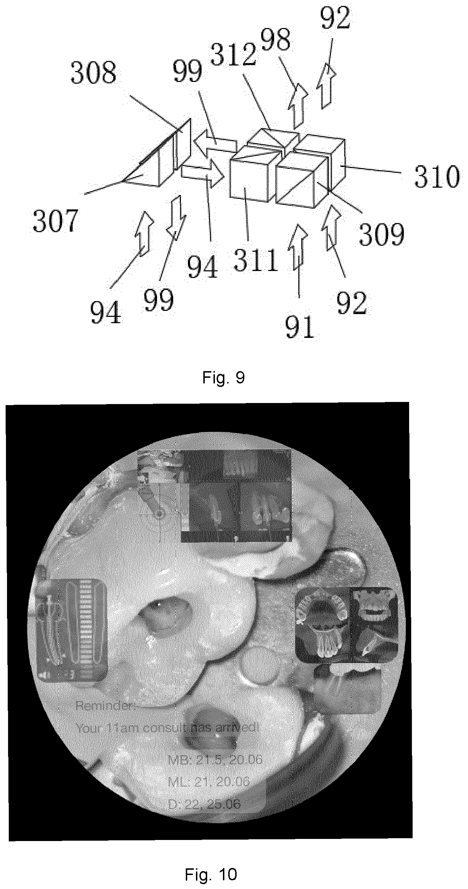

In this embodiment, on the basis of Embodiment VII, in the superimposition lens set 3, a fourth transverse beam splitter prism 311 is added between the second reflector 307 and the fourth longitudinal beam splitter prism 309, and a fifth transverse beam splitter prism 312 is added between the third transverse beam splitter prism 310, and the third reflector 308 and the fourth transverse beam splitter prism 311. As shown in FIG. 9, the additional information beam 94 reflected by the second reflector 307 is split laterally by the fourth transverse beam splitter prism 311. One part of the additional information beam 94 enters the fourth longitudinal beam splitter prism 309 along the original optical path, and the other part of the additional information beam 94 is deflected by 90° and enters the fifth transverse beam splitter prism 312. The part of the second incident beam 92 split laterally by the third transverse beam splitter prism 310 is superimposed with the part of the additional information beam 94 entering the fifth transverse beam splitter prism 312 through the fifth transverse beam splitter prism 312 to form a fifth superimposed beam 99. The fifth superimposed beam is reflected by the third reflector 308 and exits downward. The fifth superimposed beam 99 exiting downward can be collected by the image device 7 so as to provide an image for the image device 7. In this case, the image obtained by the image device 7 is a superimposed image.

It should be noted that in the above embodiments, an optical zoom system may also be arranged in the imaging lens set 62. By adjusting the optical zoom system, the size of the image formed by the additional information beam 94 can be adjusted.

Moreover, a filter apparatus 8 may be arranged between the superimposition lens set 3 and the binocular tube 5. The filter apparatus 8 is mainly configured to adjust the brightness of the additional information image to be matched with the brightness of the optical image of the surgical microscope, which is convenient for observation. Besides, an optical filter may be arranged to remove or reduce the part of spectrum harmful to human eyes, such as reducing blue light hazard. A polarization filter or a spatial filter apparatus, such as a diaphragm with different clear apertures, may also be arranged as needed. A plurality of filter apparatuses 8 may be switched by rotating the turntable, pushing and pulling, or the like.

The above embodiments are exemplary descriptions of the structure of the surgical microscope in the surgical microscope diagnosis and treatment system. Specific contents of the surgical microscope diagnosis and treatment system will be described below.

### Embodiment IX

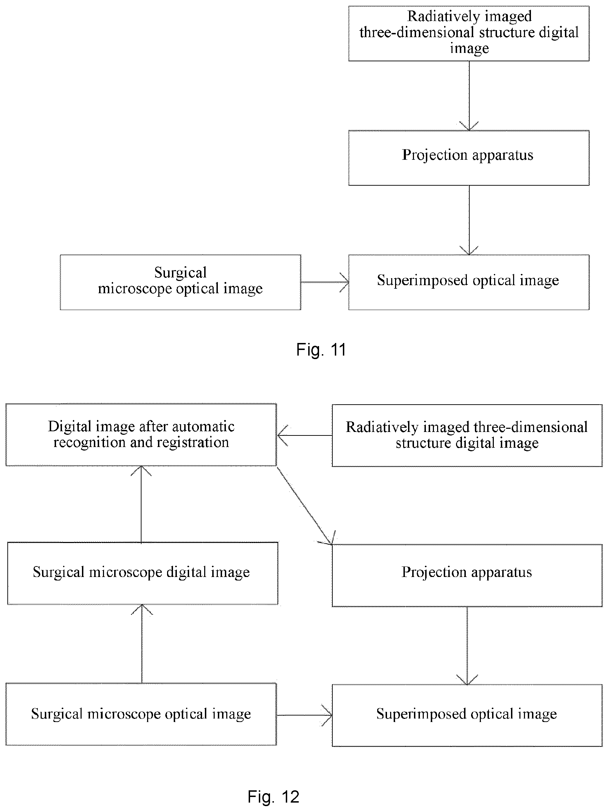

The surgical microscope diagnosis and treatment system comprises a microscopic observation module, a storage module and an enhanced information image injection module. The microscopic observation module is configured to observe a target object to be observed. The microscopic observation module is any surgical microscope in the above embodiments. A radiatively imaged three-dimensional structure digital image of the target object is imported into the storage module by means of external input or retrieval. The radiatively imaged three-dimensional structure digital image is preferably a CBCT digital image. All the following embodiments describe the technical solution in an example of the CBCT digital image. The enhanced information image injection module is configured to project the radiatively imaged three-dimensional structure digital image to an edge of an observation field of the microscopic observation module in a manner of an optical image, so as to be superimposed on an optical image under a microscope in the observation field of the microscopic observation module to form a superimposed optical image, as shown in FIG. 10, so as to provide reference for the operator in real time. The system flow is shown in FIG. 11. The enhanced information image injection module is the projection apparatus 6 in the above embodiments. Additional digital information needs to be integrated, transmitted to the projection display unit 61 by means of wired HDMI, SDI, Internet port, wireless wifi, Bluetooth, etc., and converted to an optical image which is then outputted.

The CBCT digital image is controllable to be on or off, so that the operator can choose to view a layered two-dimensional image or a 3D image according to needs. The operator can determine the internal structure of the tissue under the microscope by choosing to view the specific layered two-dimensional image. In this embodiment, the operator needs to manually compare and register the CBCT digital image with the optical image under the microscope.

### Embodiment X

In the surgical microscope diagnosis and treatment system of this embodiment, on the basis of Embodiment IX, a 3D imaging module and an image recognition and processing module are added. The 3D imaging module is configured to collect the optical image under the microscope in the observation field of the microscopic observation module in real time and convert the optical image under the microscope into a three-dimensional digital image. The image recognition and processing module is configured to recognize biological characteristics in the three-dimensional digital image, automatically compare and register the CBCT digital image with the three-dimensional digital image by means of comparison of biological characteristics, and transmit the CBCT digital image matched with the three-dimensional digital image to the enhanced information image injection module, and finally, the CBCT digital image is projected into the observation field of the microscopic observation module. The system flow is shown in FIG. 12.

The surgical microscope diagnosis and treatment system of this embodiment further comprises a detection module. The detection module is configured to respectively detect a focusing position of the zoom large objective lens (i.e., the large objective lens set 4) and a magnification of the zoom system (i.e., the zoom lens set 2) in the surgical microscope. The detection module is preferably a position sensor. That is, position sensors are respectively added to the surgical microscope at the positions of the zoom large objective lens and the zoom system. The image recognition and processing module determines a depth position of the CBCT digital image according to the focusing position of the zoom large objective lens detected by the detection module. The image recognition and processing module determines a depth range of a current layer area displayed by the CBCT digital image according to the magnification of the zoom system detected by the detection module. The focal depth position of the surgical microscope is automatically registered with the layered depth of CBCT, that is, when a plane structure is observed under the microscope, the CBCT digital image automatically shows the CT digital image of the current layer (or current layer area).

### Embodiment XI

In the surgical microscope diagnosis and treatment system of this embodiment, on the basis of Embodiment X, the CBCT digital image is displayed in superposition with the optical image under the microscope in the observation field of the microscopic observation module. A transparency of the CBCT digital image is adjustable.

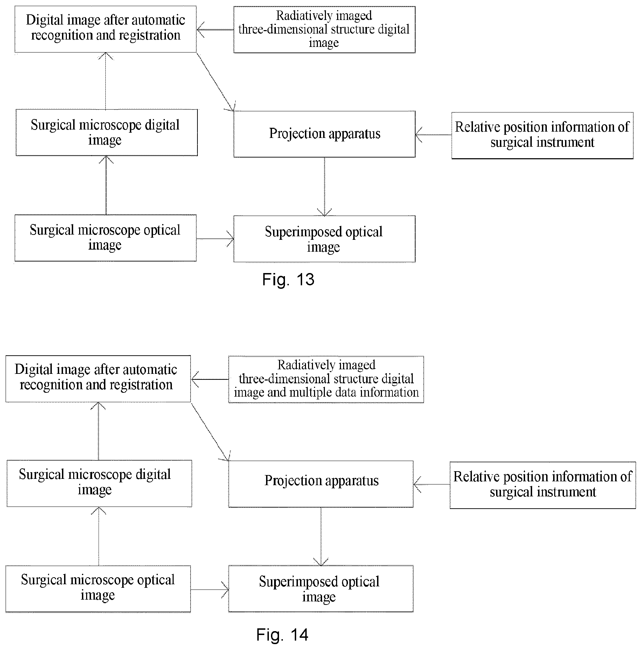

The surgical microscope diagnosis and treatment system of this embodiment further comprises a positioning and navigation-based detection module. The positioning and navigation-based detection module is mounted on a surgical instrument. The surgical instrument is, for example, a dental handpiece. The positioning and navigation-based detection module is configured for real-time detection of depth and spatial position data of the surgical instrument. The image recognition and processing module compares the depth and spatial position data collected by the positioning and navigation-based detection module with the biological characteristics in the three-dimensional digital image, such as the position and shape of the root canal orifice, the depth and trend of the root canal, to obtain real-time relative position data between the surgical instrument and the target object, and transmits the real-time relative position data to the enhanced information image injection module, and then the real-time relative position data is projected to a set position of the observation field of the microscopic observation module. The system flow is shown in FIG. 13. Moreover, state data of the surgical instrument, such as speed and torque of the handpiece, may be introduced, or projected to the set position of the observation field of the microscopic observation module by means of the enhanced information image injection module. When the position sensors arranged at the zoom large objective lens and the zoom system detect changes, real-time data of the surgical instrument is automatically projected to the set position of the observation field of the microscopic observation module, so that the doctor can confirm the operation in time, which improves the efficiency. The depth and spatial position data of the surgical instrument, and the state data of the surgical instrument can be stored in the storage module in real time.

### Embodiment XII

In the surgical microscope diagnosis and treatment system of this embodiment, on the basis of any of Embodiment IX to Embodiment XI, more related data is introduced. For example, information such as patient information data, apex locator data, intraoral scanner data, electronic periodontal probe data and pulp vitality data can all be stored into the storage module by means of external input or retrieval. Each data is respectively configured with a display switch and controllable to be on or off separately, so that an operator can project required data to a set position of the observation field of the microscopic observation module according to needs by means of the enhanced information image injection module. The system flow is shown in FIG. 14.

Each data may be projected to the set position of the observation field of the microscopic observation module in a manner of text and symbols, data tables, two-dimensional curves or three-dimensional topographic maps. Each data is displayed in a split screen in the observation field of the microscopic observation module or switched to display in a same window, and a display transparency of each data and a size and a position of the display window of each data are adjustable.

Patient information data: This comprises basic information of the patient, restriction reminders, etc., as well as monitoring information, such as blood pressure, oxygen saturation, etc.

Apex locator data: Accurate measurement of the working length of a root canal is the basic condition for successful root canal treatment. The preparation and filling of the end position for teeth with different conditions vary, and a margin of error of ±0.5 mm needs to be ensured, so the electronic apex locator is used. The data of the electronic apex locator can be selectively displayed to provide reference.

Intraoral scanner data: High-resolution three-dimensional form of structure of the oral cavity.

Electronic periodontal probe data: It is an indisputable fact in international stomatology that periodontal health is the foundation of a healthy oral cavity. Periodontal probing is an important method for oral basic diagnosis. The measurement of the depth and attachment level of the periodontal pocket with a periodontal probe is currently the main method for clinical assessment of the degree of periodontal destruction, and it is also the clinical basis for determining changes in periodontal disease. A Florida Probe system can automatically measure the depth and attachment level of the periodontal pocket, and attached gingival width of the patient under the operation of one medical worker, and record the complete dentition, tooth looseness, gingival bleeding and suppuration, furcation involvement, plaque distribution and other indicators that can reflect the severity of periodontal disease and prognosis. The system comes with a risk factor assessment function to effectively assess the risk of the patient's condition, which helps the doctor to be able to objectively develop a targeted treatment plan for the patient.

Pulp vitality data: Pulp is located in the pulp cavity enclosed by dentin and connected to the periapical tissue through a narrow apical foramen. The pulp cannot be seen directly, which makes it impossible to visually determine its specific state in clinic. There are abundant nerves distributed in the pulp, which can sense external stimuli. Clinically, the pulp vitality is determined by stimulating nerve fibers of the pulp by temperature and electricity, which helps the doctor to choose whether to remove necrotic pulp completely or to perform amputation to preserve healthy parts.

### Embodiment XIII

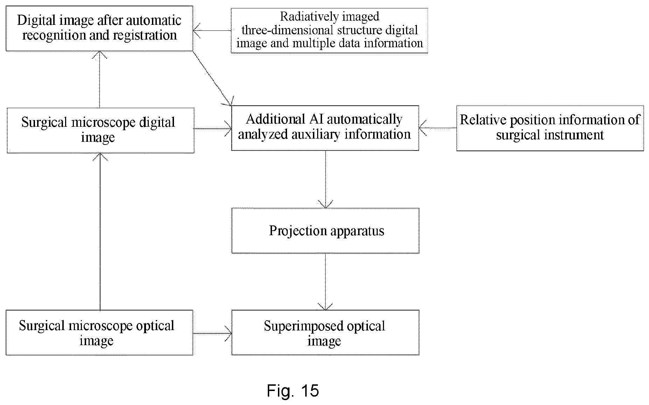

In the surgical microscope diagnosis and treatment system of this embodiment, on the basis of any of Embodiment IX to Embodiment XII, an Al auxiliary analysis module is added to realize Al auxiliary diagnosis and display. The Al auxiliary analysis module is controllable to be on or off by means of a master switch or a separate function switch, so that the operator can choose to enable or disenable the corresponding Al auxiliary function according to needs. The Al auxiliary analysis module is configured to analyze a three-dimensional digital image collected by a 3D imaging module, identify lesions of the target object and leave a mark or reminder on the target object according to the lesions, and also configured to comprehensively analyze all the data stored in the storage module to generate additional Al auxiliary information, so that the operator can project the Al auxiliary information to the set position of the observation field of the microscopic observation module by means of the enhanced information image injection module. The system flow is shown in FIG. 15.

For example, only the three-dimensional digital image collected by the 3D imaging module is analyzed:
Analysis is performed based on the digital image under the microscope under normal white light illumination to distinguish lesions of the oral cavity and teeth, such as caries, cracked teeth, plaque, discoloration, oral cancer, etc., and a mark or a reminder is left. The mark may be left by means of different colors of text, frames, edge lines, arrows, staining or a combination thereof, or in combination with prompt sound. For suspected lesions that need further examination, the mark is automatically left, and the operator is reminded to switch to the corresponding working mode, such as fluorescence detection mode in different wavelengths, polarization working mode or different filter modes, to perform the examination. For the suspected lesions that need to switch different working modes for further examination, the Al module may automatically control the microscope to switch modes and obtain the image of the corresponding mode for further analysis. For the specific implementation of mode switching, reference may be made to the patent CN211741708U, and details will not be described here.

Moreover, other imported data is analyzed comprehensively: The patient information (age, gender, blood pressure, oxygen saturation, past medical history, etc.), and information of CBCT, electronic apex locator, electronic periodontal probe, pulp vitality data and the like are analyzed comprehensively, the tooth state is assessed based on an expert system, and feasible treatment plans and steps are provided as suggestion. In combination with the spatial position and depth information of the surgical instrument, the size, depth and speed during tooth grinding are provided as instructions or reminders to improve the standardization of operation.

In the surgical microscope diagnosis and treatment system of this embodiment, a camera module, such as a video camera, etc., may be added to obtain expression image data of the patient in real time. The Al auxiliary analysis module analyzes the expression image data collected by the camera module, determines a comfort level of the patient, and projects the real-time projection to the set position of the observation field of the microscopic observation module by means of the enhanced information image injection module, so that the operator can know the state of the patient at any time.

In this specification, the term "comprise", "comprise" or any other variation thereof is intended to cover non-exclusive inclusion, which comprises not only those listed elements, but also other elements not explicitly listed.

In this specification, the directional terms such as front, back, up and down are defined based on the positions of the parts in the drawings and between the parts, and are only for the clarity and convenience of expressing the technical solution. It should be understood that the use of the directional terms shall not limit the scope of this application.

In the case of no conflict, the above embodiments and features in the embodiments herein can be combined with each other.

The above description is only the preferred embodiments of the present invention and is not intended to limit the present invention. Any modifications, equivalent substitutions and improvements made within the spirit and principles of the present invention shall be comprised within the protection scope of the present invention.

## Claims

1. A surgical microscope diagnosis and treatment system, comprising a microscopic observation module, a storage module and an enhanced information image injection module, wherein
the microscopic observation module is configured to observe a target object to be observed;
the storage module stores a radiatively imaged three-dimensional structure digital image of the target object; and
the enhanced information image injection module is configured to project the radiatively imaged three-dimensional structure digital image into an observation field of the microscopic observation module in a manner of an optical image, so as to be superimposed on an optical image under a microscope in the observation field of the microscopic observation module to form a superimposed optical image.

2. The surgical microscope diagnosis and treatment system according to claim 1, wherein the radiatively imaged three-dimensional structure digital image is controllable to be on or off, so that an operator chooses to view a layered two-dimensional image or a 3D image according to needs.

3. The surgical microscope diagnosis and treatment system according to claim 2, further comprising a 3D imaging module and an image recognition and processing module, wherein the 3D imaging module is configured to collect the optical image under the microscope in the observation field of the microscopic observation module in real time and convert the optical image under the microscope into a three-dimensional digital image; and the image recognition and processing module is configured to recognize biological characteristics in the three-dimensional digital image, perform comparison of biological characteristics and transmit the radiatively imaged three-dimensional structure digital image matched with the three-dimensional digital image to the enhanced information image injection module, and then the radiatively imaged three-dimensional structure digital image is projected into a set area in the observation field of the microscopic observation module.

4. The surgical microscope diagnosis and treatment system according to claim 3, further comprising a detection module, wherein a zoom large objective lens and a zoom system are arranged in the microscopic observation module; the detection module is configured to respectively detect a focusing position of the zoom large objective lens and a magnification of the zoom system; the image recognition and processing module determines a depth position of the radiatively imaged three-dimensional structure digital image according to the focusing position of the zoom large objective lens detected by the detection module; and the image recognition and processing module determines a depth range of a current layer area displayed by the radiatively imaged three-dimensional structure digital image according to the magnification of the zoom system detected by the detection module.

5. The surgical microscope diagnosis and treatment system according to claim 3, wherein the radiatively imaged three-dimensional structure digital image is projected to an edge of the observation field of the microscopic observation module, or the radiatively imaged three-dimensional structure digital image is displayed in superposition with the optical image under the microscope in the observation field of the microscopic observation module, and a transparency of the radiatively imaged three-dimensional structure digital image is adjustable.

6. The surgical microscope diagnosis and treatment system according to claim 5, further comprising a positioning and navigation-based detection module, wherein the positioning and navigation-based detection module is mounted on a surgical instrument; the positioning and navigation-based detection module is configured for real-time detection of depth and spatial position data of the surgical instrument; and the image recognition and processing module compares the depth and spatial position data collected by the positioning and navigation-based detection module with the biological characteristics in the three-dimensional digital image to obtain real-time relative position data between the surgical instrument and the target object, and transmits the real-time relative position data to the enhanced information image injection module, and then the real-time relative position data is projected to a set position of the observation field of the microscopic observation module.

7. The surgical microscope diagnosis and treatment system according to claim 6, wherein the depth and spatial position data of the surgical instrument and state data of the surgical instrument are stored in the storage module in real time, and the state data of the surgical instrument is capable of being transmitted to the enhanced information image injection module and then projected to the set position of the observation field of the microscopic observation module.

8. The surgical microscope diagnosis and treatment system according to claim 1, wherein the storage module also stores patient information data, apex locator data, intraoral scanner data, electronic periodontal probe data and pulp vitality data, and each data is respectively controllable to be on or off, so that an operator projects required data to a set position of the observation field of the microscopic observation module according to needs by means of the enhanced information image injection module.

9. The surgical microscope diagnosis and treatment system according to claim 8, wherein each data is projected to the set position of the observation field of the microscopic observation module in a manner of text and symbols, data tables, two-dimensional curves or three-dimensional topographic maps, each data is displayed in a split screen in the observation field of the microscopic observation module or switched to display in a same window, and a display transparency of each data and a size and a position of the display window of each data are adjustable.

10. The surgical microscope diagnosis and treatment system according to claim 9, further comprising an Al auxiliary analysis module, wherein the Al auxiliary analysis module is controllable to be on or off, so that the operator chooses to turn on or off an Al auxiliary function according to needs; and the Al auxiliary analysis module is configured to analyze a three-dimensional digital image collected by a 3D imaging module, identify lesions of the target object and leave a mark or reminder on the target object according to the lesions, and also configured to comprehensively analyze all the data stored in the storage module to generate additional Al auxiliary information, and then the Al auxiliary information is projected to the set position of the observation field of the microscopic observation module by means of the enhanced information image injection module.

11. The surgical microscope diagnosis and treatment system according to claim 10, further comprising a camera module, wherein the camera module is configured to collect expression image data of a patient; and the Al auxiliary analysis module analyzes the expression image data collected by the camera module, determines a comfort level of the patient, and projects a real-time projection to the set position of the observation field of the microscopic observation module by means of the enhanced information image injection module.

12. The surgical microscope diagnosis and treatment system according to claim 1, wherein the microscopic observation module comprises a surgical microscope; the enhanced information image injection module comprises a projection apparatus (6); the projection apparatus (6) is capable of projecting an additional information beam (94); and the additional information beam (94) and one or two incident beams in the surgical microscope are superimposed and then enter a binocular tube (5) of the surgical microscope to form the superimposed optical image.

13. The surgical microscope diagnosis and treatment system according to claim 12, wherein the projection apparatus (6) comprises a projection display unit (61) and an imaging lens set (62), the projection display unit (61) being capable of projecting an additional optical image (93), and the additional optical image (93) being converted into parallel beams by the imaging lens set (62) to form the additional information beam (94).
